# FASCICULE DE BREVET EUROPEEN

(11) **EP 2 595 563 B1**
(45) Date de publication et mention de la délivrance du brevet: **01.02.2017**
(21) Numéro de dépôt: 11735866.3
(22) Date de dépôt: 18.07.2011
(51) Int. Cl.: A61C 7/36, A61F 5/56

(54) **ORTHESE COMPRENANT UNE GOUTTIERE SUPERIEURE ET UNE GOUTTIERE INFERIEURE, ET DES MOYENS DE LIAISON REGLABLES EN POSITION**
ORTHESE MIT EINEM OBEREN UND UNTEREN TRAY SOWIE EINER VERBINDUNGSVORRICHTUNG MIT EINSTELLBARER POSITIONIERUNG
ORTHOSIS COMPRISING AN UPPER TRAY AND A LOWER TRAY, AND ADJUSTABLE POSITIONING MEANS

(30) Priorité: 20.07.2010 FR 1003041
(43) Date de publication de la demande: 29.05.2013
(73) Titulaire: Medventiv, 35700 Rennes (FR)
(72) Inventeur: KLEIN, Dominico, F-35700 Rennes (FR); PERLADE, Dominique, F-44360 Vigneux De Bretagne (FR)
(74) Mandataire: Vidon Brevets & Stratégie
(86) Numéro de dépôt international: PCT/EP2011/062284
(87) Numéro de publication internationale: WO 2012/010565

(56) Documents cités:
- EP-A1- 0 801 937
- EP-A1- 1 867 309
- DE-A1- 10 042 049
- US-A1- 2006 174 897
- US-B1- 6 767 207

## Description

L'invention concerne le domaine de la conception et de la fabrication des dispositifs orthopédiques et/ou orthodontiques. Plus précisément, l'invention concerne une orthèse préfabriquée de type amovible.

La spécialité d'ODF (orthopédie dento-faciale), communément appelée orthodontie vise à la prévention, à la correction des malpositions dentaires et à l'établissement d'un contact inter-dentaire optimal. Elle s'applique aussi bien à rétablir l'esthétique de la face par le biais des maxillaires (appelées couramment « mâchoires ») qu'à corriger les fonctions comme la respiration, la mastication et l'élocution.

L'orthodontie traite les malpositions des dents et a pour dessein de les positionner en harmonie esthétique et fonctionnelle sur les arcades dentaires.

Après correction des malpositions par des appareils fixes notamment sous formes d'attaches collées sur la couronne des dents, la denture est soumise à un environnement musculaire (les arcades dentaires se trouvent au centre d'un mur musculaire interne : la langue et d'un autre mur musculaire externe latéral : les joues formées par les muscles buccinateurs et d'un mur musculaire antéro-externe les muscles des lèvres et en particulier l'orbiculaire) qui évolue et, par conséquent, ne garantit pas la stabilité et le résultat de la correction.

Une phase de maintien du résultat est alors indispensable : il s'agit de la contention.

La contention est assurée soit par des dispositifs fixes collés sur les dents, soit par des dispositifs amovibles.

Parmi les dispositifs amovibles, on distingue les gouttières placées indépendamment sur chaque arcade dentaire, ou les gouttières double, préfabriquées et prenant en charge les deux arcades dentaires simultanément dans un rôle « positionneur ».

L'invention concerne les orthèses de type amovibles, à gouttière double, pour la contention post traitement orthodontique.

On note qu'une orthèse selon l'invention peut également être utilisée, dans des cas simples et bénins, pour le traitement du syndrome d'apnée hypopnées du sommeil (SAHS), ou encore le traitement visant à prévenir des problèmes d'origine fonctionnelle : il s'agira alors d'appareil d'interception et de guidage. Dans le domaine de l'invention, plusieurs types d'orthèse à double gouttières ont été proposés par l'art antérieur.

On connaît notamment une orthèse telle que décrite par le document de brevet FR-2 820 307, formée de deux gouttières en forme générale de U, à savoir une gouttière inférieure dite mandibulaire et une gouttière supérieure dite maxillaire, qui présentent chacune des alvéoles dentaires individuelles et comportant des éléments en saillie de formes complémentaires, aptes à venir s'engager les uns dans les autres pour assurer la liaison des gouttières. Les éléments en saillie sont de type à tenon mortaise, de préférence à queue d'aronde.

Ainsi, il est possible de fixer la gouttière inférieure avec la gouttière supérieure avec un réglage possible dans le sens antéro-postérieur.

Cette solution présente notamment les inconvénients suivants :
- les éléments en saillie à engagement par déformation élastique ne permettent pas une liaison fiable et l'utilisateur peut par conséquent désengager la gouttière inférieure de la gouttière supérieure, puis les remettre en place dans une position éventuellement inappropriée ;
- les éléments en saillie par assemblage simple ou par assemblage collé n'offrent pas la garantie que les gouttières supérieure et inférieure resteront solidaires de façon pérenne lorsque le dispositif sera mis en bouche, rendant ainsi l'appareil de contention inopérant ;
- dans l'hypothèse d'une fabrication en grande série par injection plastique, le système à tenon mortaise peut s'avérer coûteux à industrialiser.

On connaît une autre orthèse décrite par le document de brevet publié sous le numéro WO-03/034957, qui décrit une orthèse intra-orale comprenant :
- une gouttière supérieure et une gouttière inférieure destinées à revêtir respectivement une denture d'une mâchoire supérieure et une denture d'une mâchoire inférieure ;
- deux tirants retenant les gouttières, ces tirants étant d'une longueur telle que la mâchoire inférieure est maintenue dans une position avancée par rapport à la mâchoire supérieure.

Dans cette solution, les tirants présentent des moyens permettant le réglage de leur longueur. Plusieurs solutions sont proposées pour la réalisation de ses tirants de longueur variable, à savoir :
- les tirants présentent deux passages filetés dans lesquels se visse une tige dont les extrémités présentent un pas inversé, un écrou étant placé au centre de la tige ;
- les tirants comprennent un cylindre dans lequel coulisse sous l'action d'une pression hydraulique une tige dont l'extrémité comprend un piston ;
- les tirants comprennent deux barrettes coulissant l'une dans l'autre, chacun étant pourvus de perçage.

De telles solutions présentent notamment les inconvénients suivants :
- les tirants sont des pièces mécaniques de petites dimensions, demandant un usinage relativement précis, ce qui tend à rendre la réalisation des tirants coûteuse ;
- les tirants peuvent présenter des parties saillantes, susceptibles de causer de petites coupures à la personne qui porte l'orthèse.

On connaît aussi une orthèse décrite dans le document de brevet publiée sous le numéro EP0801937A1, qui décrit un appareil intra-oral qui peut notamment être composé de deux gouttières reliées par un mécanisme de réglage par vissage ou par emboîtement et vissage. Cependant, l'orthèse selon cette solution est moins facile à régler et le mécanisme de réglage proposé est complexe à fabriquer.

L'invention a notamment pour objectif de pallier les inconvénients de l'art antérieur.

Plus précisément, l'invention a pour objectif de proposer une orthèse dans laquelle les moyens reliant la gouttière inférieure à la gouttière supérieure peuvent être fabriqués de façon peu coûteuse.

L'invention a également pour objectif de fournir une telle orthèse qui offre un niveau satisfaisant de confort et de qualité de traitement au patient.

Ces objectifs, ainsi que d'autres qui apparaîtront par la suite, sont atteints grâce à l'invention qui a pour objet une orthèse comprenant :
- une gouttière supérieure et une gouttière inférieure destinées à revêtir respectivement une denture d'une mâchoire supérieure et une denture d'une mâchoire inférieure ;
- des moyens de liaison reliant les gouttières,
caractérisée en ce que ladite gouttière supérieure présente au moins une première embase fixe, en ce que ladite gouttière inférieure présente au moins une deuxième embase fixe, et en ce que lesdits moyens de liaison incluent lesdites première et deuxième embases, celles-ci présentant des complémentaires de telle sorte que l'une des embase forme un guidage en coulissement de l'autre, selon un positionnement à effet propulsif réglable.

Ainsi, grâce à l'invention, l'orthèse est réglée de façon appropriée aux besoins du patient, sans qu'il soit possible pour celui-ci, de modifier ce réglage par inadvertance comme cela va apparaître plus clairement par la suite.

En outre, les moyens de liaison d'une orthèse selon l'invention peuvent être fabriqués simplement, de façon peu coûteuse, dans la mesure où ils sont de longueur fixe et ne comprennent donc pas de parties mobiles entre elles.

Les moyens de liaison sont également peu coûteux à réaliser dans la mesurer où les moyens de réglage de leur position entre les deux gouttières sont intégrés à l'une au moins des embases intégrées aux gouttières, et non directement sur des moyens de liaison supplémentaires tels que ceux mentionnés en référence à l'art antérieur.

Selon un premier mode de réalisation, ladite première embase présente une coulisse, ladite deuxième embase constituant un coulisseau susceptible de coulisser dans ladite coulisse.

Selon un deuxième mode de réalisation, ladite deuxième embase présente une coulisse, ladite première embase constituant un coulisseau susceptible de coulisser dans ladite coulisse.

Dans l'un ou l'autre cas, ladite première embase et ladite deuxième embase sont destinées à être solidarisées par au moins une vis, cette vis étant préférentiellement auto-taraudeuse. En outre, une telle vis est préférentiellement "auto-freinée" grâce à un pas de vis optimal.

Selon une première variante de réalisation, ladite première embase présente un unique passage de vis tandis que ladite deuxième embase présente au moins deux passages de vis destinés à être l'un ou l'autre placé en coïncidence avec l'unique passage de la première embase.

Selon une deuxième variante de réalisation, ladite deuxième embase présente un unique passage de vis tandis que ladite première embase présente au moins deux passages de vis destinés à être l'un ou l'autre replacé en coïncidence avec l'unique passage de la deuxième embase.

Selon une solution avantageuse, chaque embase comprend une base formant un élément de couplage avec l'une des gouttières, ladite coulisse ou ledit coulisseau s'étendant à partir de ladite base.

Selon un mode de réalisation particulier, l'une desdites embases présente l'une et/ou l'autre des caractéristiques suivantes :
- elle présente une série de passages de vis permettant un réglage du positionnement à effet propulsif sur une course de 10 mm ;
- elle présente une série de passages de vis permettant un réglage du positionnement à effet propulsif avec un pas de 2 mm.

Selon une autre caractéristique de l'invention, ladite gouttière supérieure présente une première surface de contact et ladite gouttière inférieure présente une deuxième surface de contact en appui contre ladite première surface de contact selon un plan de contact de pente correspondante à la direction de réglage du positionnement propulsif des moyens de liaison.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une vue en perspective d'une orthèse selon l'invention ;
- les figures 2 et 3 sont des vues éclatées d'une orthèse selon l'invention, respectivement en perspective et de côté ;
- les figures 4 et 5 sont des vues des moyens de liaison inter-arcade d'une orthèse selon l'invention, respectivement en perspective et de côté.

Tel qu'indiqué précédemment, le principe de l'invention réside dans le fait de proposer une orthèse dans laquelle les moyens de liaison reliant la gouttière supérieure à la gouttière inférieure sont réglables en position pour procurer un effet propulsif variable (réglable), ceci par l'intermédiaire de moyens de réglage intégrés sur l'une au moins des deux gouttières.

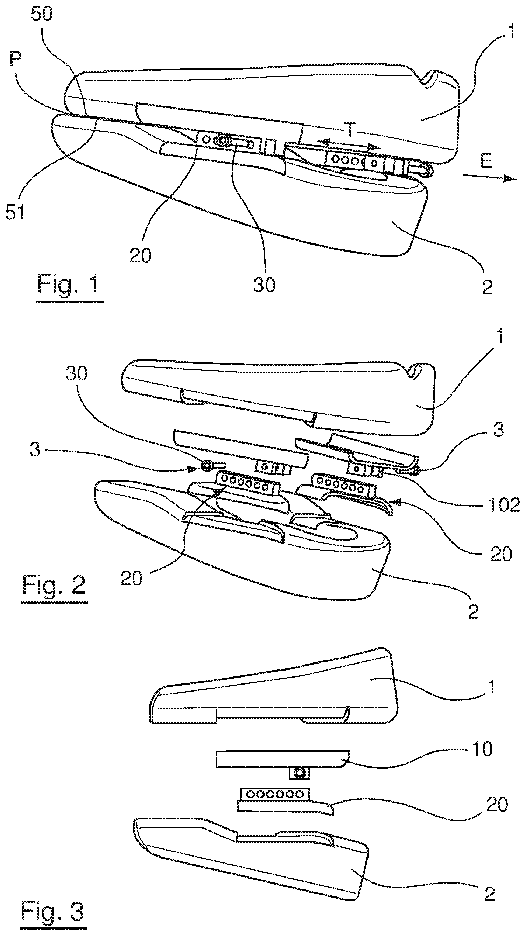

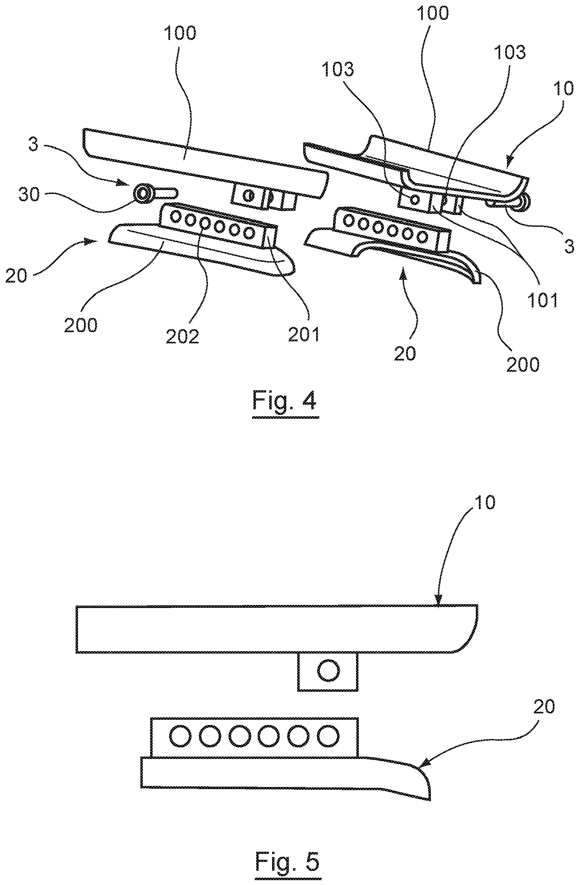

En référence aux figures 1 à 4, une orthèse selon l'invention comprend :
- une gouttière supérieure 1 destinée à revêtir une denture d'une mâchoire supérieure ;
- une gouttière inférieure 2 destinée à revêtir une denture d'une mâchoire inférieure ;
- des moyens de liaison inter-arcade reliant les gouttières, décrits plus en détails par la suite.

Selon le principe de l'invention, l'orthèse est conçue de la façon suivante :
- la gouttière supérieure 1 présente une première embase fixe 10 ;
- la gouttière inférieure 2 présente une deuxième embase fixe 20 ;

Le réglage du positionnement des moyens de liaison en vue de faire varier l'effet propulsif est permis par l'intermédiaire de moyens de réglage intégrés à l'une au moins des deux embases.

Les moyens de liaison inter-arcade sont constitués par l'association des embases supérieure et inférieure, et sont prévus pour un positionnement en normo-occlusion ou en propulsion.

Le réglage du positionnement des moyens de liaison en vue de faire varier l'effort propulsif est permis par l'intermédiaire de liaisons glissières résultant des formes complémentaires des embases supérieures et des embases inférieures.

Tel que cela apparaît sur les figures 2 à 5, l'embase supérieure 10 comprend :
- une base 100 formant élément de couplage par l'intermédiaire duquel l'embase supérieure est solidarisée à la gouttière supérieure ;
- une coulisse 102 formée par deux bossages 101 écartés l'un de l'autre et dont les parois tournées l'une vers l'autre délimitent la coulisse 102.

Les bossages 101 présentent chacun un orifice traversant 103, les orifices traversant 103 des deux bossages d'une même embase étant coaxiaux.

La deuxième embase comprend quant à elle :
- une base 200 formant élément de couplage, par l'intermédiaire duquel l'embase est solidarisée à la gouttière supérieure 2 ;
- un coulisseau 201, longiforme, et présentant sur sa longueur une pluralité d'orifices traversants 202.

La coulisse 102 et le coulisseau 201 présentent des formes complémentaires de telle sorte que l'embase supérieure forme un moyen de guidage en translation du coulisseau 201 entre les bossages 101 de la première embase.

On note que, selon des variantes envisageables, la position supérieure/inférieure des différents organes peut varier, par exemple :
- le coulisseau peut être prévu sur l'embase de la gouttière supérieure, la coulisse étant alors prévue sur l'embase de la gouttière inférieure ;
- les bossages formant la coulisse peuvent être longiformes et présenter une pluralité de trous (le coulisseau pouvant alors présenter une longueur réduite, avec éventuellement un seul orifice traversant).

A titre indicatif, les gouttières 1, 2 sont réalisées soit en élastomère soit en thermoplastique ; les matériaux utilisés sont biocompatibles grade médical. Ces matériaux peuvent être de différentes duretés.

Les embases 10, 20 sont réalisées en matériaux thermoplastiques biocompatibles grade médical, et sont directement intégrées aux gouttières pour former avec elles un ensemble d'un seul tenant.

Les moyens de liaison sont en outre conçus de façon à présenter un faible encombrement, et en particulier un faible débord lingual.

Comme indiqué précédemment, les moyens de liaison inter-arcade sont constitués par l'association des première et deuxième embases, ceci préférentiellement par un assemblage anti-desserrage à l'aide de micro-vis à pas de vis optimal, dévissables par un tournevis spécial. Les vis 3 utilisées sont en matériau métallique biocompatible grade médical.

Pour procéder au réglage du positionnement de la gouttière supérieure par rapport à la gouttière inférieure, on engage le coulisseau de l'une des embases dans la coulisse de l'autre embase. On fait coulisser le coulisseau dans la coulisse de façon à obtenir le réglage voulu, ceci en plaçant l'un des orifices 202 de la deuxième embase (ou de la première embase selon le cas) avec l'orifice (ou l'un des orifices selon le cas) de la première embase (ou de la deuxième embase selon le cas).

On comprend, bien entendu, que chaque orifice traversant, que ce soit celui des bossages formant la coulisse ou que ce soit celui ou ceux du coulisseau, constitue un passage pour une vis de fixation 3.

Préférentiellement, les bossages 101 formant la coulisse 102 sont espacés l'un de l'autre de façon à former un logement apte à épouser le coulisseau de l'autre embase. Plus précisément, la liaison glissière entre les deux embases est obtenue en faisant coïncider concomitamment les première et deuxième embases (coulisse et coulisseau) de telle sorte que le coulisseau vient se caler dans la coulisse.

Le positionnement est ensuite fixé par les vis de fixation qui sont introduites en premier lieu dans le bossage externe de la première embase, la vis venant traverser l'orifice traversant choisi du coulisseau, puis étant ensuite vissée par auto-taraudage dans le bossage interne de la coulisse.

Le diamètre de l'orifice traversant du bossage interne est donc adapté au diamètre de la vis de fixation, ceci en étant inférieur à celui-ci de telle sorte que la vis procède à un auto-taraudage du bossage interne.

Les vis de fixation sont donc introduites de l'extérieur vers l'intérieur de l'orthèse telle que l'indique la position de la tête 30 des vis 3 sur les figures 1 à 3.

Selon une disposition préférée, les orifices 202 sont disposés de la façon suivante :
- ils permettent un réglage du positionnement à effet propulsif des moyens de liaison 3 sur une course de 10 mm ;
- ils présentent entre eux une distance de 2 mm, prédéterminant ainsi le pas de réglage.

Par ailleurs, la gouttière supérieure présente une première surface de contact 50 et la gouttière inférieure 2 présente une deuxième surface de contact 51, en appui contre la première surface de contact 50 selon un plan de contact P dont la pente correspond à la direction de réglage T du positionnement à effet propulsif E des moyens de liaison 3.

En référence à la figure 1, la première surface de contact 50 de la gouttière supérieure correspond au rebord inférieur de la gouttière supérieure. La deuxième surface de contact 51 de la gouttière inférieure est prévue en partie arrière, sous forme d'une excroissance.

La translation de la gouttière inférieure par rapport à la gouttière supérieure est ainsi facilitée par les zones de contact (surfaces 50, 51), qui sont parallèles à l'axe de translation (indiqué par la double flèche T).

## Revendications

1. Orthèse comprenant :
- une gouttière supérieure (1) et une gouttière inférieure (2) destinées à revêtir respectivement une denture d'une mâchoire supérieure et une denture d'une mâchoire inférieure ;
- des moyens de liaison reliant les gouttières (1,2),
ladite gouttière supérieure (1) présentant au moins une première embase fixe (10), ladite gouttière inférieure (2) présentant au moins une deuxième embase fixe (20), lesdits moyens de liaison incluant lesdites première et deuxième embases (10, 20), ladite première embase (10) et ladite deuxième embase (20) étant destinées à être solidarisées par au moins une vis (3),
ladite orthèse étant **caractérisée en ce que** lesdites première et deuxième embases (10, 20) présentant des formes complémentaires de telle sorte que l'une des embases forme un guidage en coulissement de l'autre embase selon un positionnement à effet propulsif réglable, et **en ce que** :
- ladite première embase (10) présente un unique passage de vis (103) tandis que ladite deuxième embase présente au moins deux passages (202) de vis destinés à être l'un ou l'autre placé en coïncidence avec l'unique passage de la première embase (10);
ou
- ladite deuxième embase (20) présente un unique passage de vis tandis que ladite première embase (10) présente au moins deux passages de vis destinés à être l'un ou l'autre placé en coïncidence avec l'unique passage de la deuxième embase (20).

2. Orthèse selon la revendication 1, **caractérisée en ce que** ladite première embase (10) présente une coulisse (102), ladite deuxième embase (20) constituant un coulisseau (201) susceptible de coulisser dans ladite coulisse (102).

3. Orthèse selon la revendication 1, **caractérisée en ce que** ladite deuxième embase (20) présente une coulisse, ladite première embase constituant un coulisseau susceptible de coulisser dans ladite coulisse.

4. Orthèse selon la revendication 1, **caractérisé en ce que** ladite vis est auto-taraudeuse.

5. Orthèse selon la revendication 2 ou selon la revendication 3, **caractérisée en ce que** chaque embase (100, 200) comprend une base formant un élément de couplage avec l'une des gouttières, ladite coulisse (102) ou ledit coulisseau (201) s'étendant à partir de ladite base (100, 200).

6. Orthèse selon la revendication 1, **caractérisée en ce que** l'une desdites embases (10, 20) présente une série de passages de vis (202) permettant un réglage du positionnement à effet propulsif sur une course de 10 mm.

7. Orthèse selon la revendication 1, **caractérisée en ce que** l'une desdites embases (10, 20) présente une série de passages de vis (202) permettant un réglage du positionnement à effet propulsif avec un pas de 2 mm.

8. Orthèse selon l'une quelconque des revendications 1 à 7, **caractérisée en ce que** ladite gouttière supérieure (1) présente une première surface de contact (50) et **en ce que** ladite gouttière inférieure (2) présente une deuxième surface de contact (51) en appui contre ladite première surface de contact selon un plan de contact de pente correspondante à la direction de réglage du positionnement à effet propulsif des moyens de liaison.

## Patentansprüche

1. Orthese, umfassend:
- eine obere Schale (1) und eine untere Schale (2), die dazu bestimmt sind, ein Gebiss eines Oberkiefers und ein Gebiss eines Unterkiefers zu verkleiden;
- Verbindungsmittel, die die Schalen (1, 2) verbinden,
wobei die obere Schale (1) mindestens eine feste Basis (10) aufweist, wobei die untere Schale (2) mindestens eine zweite feste Basis (20) aufweist, wobei die Verbindungsmittel die erste und zweite Basis (10, 20) einschließen, wobei die erste Basis (10) und die zweite Basis (20) dazu bestimmt sind, durch mindestens eine Schraube (3) verbunden zu werden,
wobei die Orthese **dadurch gekennzeichnet ist, dass** die ersten und zweiten Basen (10, 20) komplementäre Formen aufweisen, so dass eine der Basen eine Gleitführung für die andere Basis mittels einer Positionierung mit einstellbarer Antriebswirkung bildet, und dass:
- die erste Basis (10) einen einzigen Schraubendurchgang (103) aufweist, während die zweite Basis mindestens zwei Schraubendurchgänge (202) aufweist, die dazu bestimmt sind, dass der eine oder der andere mit dem einzigen Schraubendurchgang der ersten Basis (10) zusammenfallend angeordnet wird;
oder
- die zweite Basis (20) einen einzigen Schraubendurchgang aufweist, während die erste Basis (10) mindestens zwei Schraubendurchgänge aufweist, die dazu bestimmt sind, dass der eine oder der andere mit dem einzigen Schraubendurchgang der zweiten Basis (20) zusammenfallend angeordnet wird.

2. Orthese nach Anspruch 1, **dadurch gekennzeichnet, dass** die erste Basis (10) eine Gleitschiene (102) aufweist, wobei die zweite Basis (20) ein Gleitstück (201) darstellt, das geeignet ist, in der Gleitschiene (102) zu gleiten.

3. Orthese nach Anspruch 1, **dadurch gekennzeichnet, dass** die zweite Basis (20) eine Gleitschiene aufweist, wobei die erste Basis ein Gleitstück darstellt, das geeignet ist, in der Gleitschiene zu gleiten.

4. Orthese nach Anspruch 1, **dadurch gekennzeichnet, dass** die Schraube eine gewindeschneidende Schraube ist.

5. Orthese nach Anspruch 2 oder 3, **dadurch gekennzeichnet, dass** jede Basis (100, 200) einen Sockel umfasst, der ein Kopplungselement mit einer der Schalen bildet, wobei sich die Gleitschiene (102) oder das Gleitstück (201) von dem Sockel (100, 200) aus erstrecken.

6. Orthese nach Anspruch 1, **dadurch gekennzeichnet, dass** eine der Basen (10, 20) eine Reihe von Schraubendurchgängen (202) aufweist, die eine Einstellung der Positionierung mit Antriebswirkung auf einem Weg von 10 mm ermöglichen.

7. Orthese nach Anspruch 1, **dadurch gekennzeichnet, dass** eine der Basen (10, 20) eine Reihe von Schraubendurchgängen (202) aufweist, die eine Einstellung der Positionierung mit Antriebswirkung auf einem Weg von 2 mm ermöglichen.

8. Orthese nach einem der Ansprüche 1 bis 7, **dadurch gekennzeichnet, dass** die obere Schale (1) eine erste Kontaktfläche (50) aufweist, und dass die untere Schale (2) eine zweite Kontaktfläche (51) aufweist, die an der ersten Kontaktfläche entlang einer Kontaktebene mit einem Gefälle entsprechend der Einstellrichtung der Positionierung mit Antriebswirkung der Verbindungsmittel anliegt.

## Claims

1. Orthosis including:
- an upper tray (1) and a lower tray (2) intended to respectively cover the teeth of an upper jaw and the teeth of a lower jaw ;
- connection means linking the trays (1,2),
**characterized in that** said upper tray (1) has at least one first fixed base (10), **in that** said lower tray (2) has at least one second fixed base (20), said connection means including said first and second bases (10, 20), said first base (10) and said second base (20) are intended to be secured by at least one screw (3),
said orthosis being **characterized in that** said first and second bases (10, 20) having complementary shapes so that one of the bases forms a sliding guide of the other, according to an adjustable positioning with advancing effect and **in that**:
- that said first base (10) has a single screw passage (103) while said second base (20) has at least two screw passages (202) intended to be one or the other placed so as to coincide with the single passage of the first base (10);
or
- said second base (20) has a single screw passage while said first base (10) has at least two screw passages intended to be one or the other placed so as to coincide with the single passage of the second base (20).

2. Orthosis according to claim 1, **characterized in that** said first base (10) has a slide track (102), said second base (20) forming a slide (201) capable of sliding in said slide track (102).

3. Orthosis according to claim 1, **characterized in that** said second base (20) has a slide track, said first base forming a slide capable of sliding in said slide track.

4. Orthosis according to claim 1, **characterized in that** said screw is self-tapping.

5. Orthosis according to claim 2 or according to claim 3, **characterized in that** each base (100, 200) includes a base forming a coupling element with one of the trays, with said slide (102) track or said slide (201) extending from said base (100, 200).

6. Orthosis according to claim 1, **characterized in that** one of said bases (10, 20) has a series of screws (202) enabling an adjustment of the positioning with advancing effect over a 10mm stroke.

7. Orthosis according to claim 1, **characterized in that** one of said bases (10, 20) has a series of screw passages (202) enabling an adjustment of the positioning with advancing effect with a 2mm pitch.

8. Othosis according to any one of claims 1 to 7, **characterized in that** said upper tray (1) has a first contact surface (50) and **in that** said lower tray (2) has a second contact surface (51) bearing against said first contact surface according to a contact plane with a slope corresponding to the direction of adjustment of the positioning with advancing effect of the connection means.
